# EUROPÄISCHE PATENTSCHRIFT

(11) **EP 1 699 429 B1**
(45) Veröffentlichungstag und Bekanntmachung des Hinweises auf die Patenterteilung: **08.10.2008**
(21) Anmeldenummer: 04790084.0
(22) Anmeldetag: 01.10.2004
(51) Int. Cl.: A61K 8/92, A61Q 13/00, C11D 3/50

(54) **EMULGIERTE PARFÜMÖLE**
EMULSIFIED PERFUME OILS
HUILES PARFUMS EMULSIFIEES

(30) Priorität: 21.11.2003 DE 10354564
(43) Veröffentlichungstag der Anmeldung: 13.09.2006
(73) Patentinhaber: Henkel AG & Co. KGaA, 40589 Düsseldorf (DE)
(72) Erfinder: RÄHSE, Wilfried, 40589 Düsseldorf (DE)
(86) Internationale Anmeldenummer: PCT/EP2004/010977
(87) Internationale Veröffentlichungsnummer: WO 2005/051339

(56) Entgegenhaltungen:
- EP-A- 0 813 862
- DE-A- 19 922 193
- FR-A- 2 637 802
- FR-A- 2 787 347
- JP-A- 5 202 387
- JP-A- 11 147 802
- US-A- 6 083 900
- US-A1- 2003 207 776
- US-B1- 6 495 501

## Beschreibung

Die Erfindung betrifft Parfumölkonzentrate in Form wässriger Emulsionen, welche einen Mindestgehalt an Parfum von 30 Gew.-% aufweisen, wobei der Gehalt der beiden Komponenten Wasser und Parfumöl(e) einen Wert von zusammen 96 Gew.-% übersteigt. Ferner betrifft die Erfindung ein Verfahren zur Herstellung solcher Mittel.

Parfumöle sind im Regelfall nur wenig wasserlöslich. Zu ihrer Einarbeitung in wässrige Zubereitungen werden daher für gewöhnlich entweder sogenannte Solubilisatoren oder Lösungsmittel, z. B. niedere Alkohole, eingesetzt. Daneben besteht die Möglichkeit der Emulgierung von Parfumölen.

Die Emulgierung von Duftstoffen ist infolge ihrer hohen Flüchtigkeit und ihrer mitunter starken Polarität recht problematisch und erfordert in der Regel die Mitverwendung wasserlöslicher organischer Lösungsmittel oder aber den Einsatz sehr hoher Mengen an Emulgatoren.

Aus dem Stand der Technik ist gemäß FR 2 787 347 A1 ein Parfümölkonzentrat in Form einer wäßrigen Emulsion bekannt, welche 97,0% einer zweiphasigen Lösung aus Wasser und Parfümöl enthält, wobei die Parfümmenge nicht angegeben ist.

In der deutschen Offenlegungsschrift DE 196 24 051 A1 werden emulgierte Duftstoffe in Form von transparenten Emulsionen, deren Tröpfchengröße zwischen 10 und 100 nm liegt, offenbart. Diese Emulsionen sind erhältlich, wenn man das Parfumöl zusammen mit einer speziellen Co-Ölkomponente unter Verwendung von Alkylglycosiden emulgiert, wobei die resultierenden Emulsionen einen Gehalt von bis zu 50 Gew.-% eines Parfumöls, von 1 bis zu 10 Gew.-% einer Co-Ölkomponente und von 1 bis zu 30 Gew.-% eines Emulgators vom Typ der Alkylglycoside aufweisen, mit der Maßgabe, daß die resultierenden Emulsionen zumindest 10% der Parfumölmenge an Co-Ölkomponente enthalten. Sind in der Emulsion beispielsweise 50 Gew.-% Parfumöl enthalten, so müssen ebenfalls mindestens 5 Gew.-% an Co-Ölkomponente enthalten sein. Bei einem Anteil von 40 Gew.-% Parfumöl sind es entsprechend mindestens 4 Gew.-% Co-Ölkomponente. Hinzu kommt in jedem Fall noch mindestens 1 Gew.-% an Emulgator.

Das bedeutet, daß die Parfumölemulsionen gemäß der DE 196 24 051 A1 einen sehr hohen Anteil an Zusatzstoffen (Emulgatoren, Co-Emulgatoren, Co-Ölkomponente) aufweisen, welche die natürliche Reinheit des Systems Parfumöl/Wasser beeinträchtigen. Im ungünstigsten Fall sind in den dort beschriebenen Parfumölemulsionen bis zu 50 Gew.-% dieser Zusatzstoffe enthalten. Im günstigsten theoretisch berechenbaren Fall sind, wenn man konzentrierte Parfumölemulsionen betrachtet, d. h. solche Emulsionen, welche einen Parfumölanteil von mindestens 30 Gew.-% bezogen auf das Mittel beinhalten, immer noch mindestens 4 Gew.-% an Zusatzstoffen in den Emulsionen enthalten, nämlich zumindest 3 Gew.-% Co-Ölkomponente und zumindest 1 Gew.-% Emulgator. Explizit offenbart wird in der DE 196 24 051 A1 jedoch nur eine einzige konzentrierte Parfumölemulsion mit einem Parfumölanteil von 30 Gew.-%. Diese Emulsion beinhaltet jedoch 11,9 Gew.-% an Zusatzstoffen, die von Wasser oder Parfumöl verschieden sind. Alle anderen dort offenbarten Emulsionen sind nicht konzentriert, weisen also einen Parfumölanteil von weniger als 30 Gew.-% auf und beinhalten dennoch mindestens 10 Gew.-% an Zusatzstoffen, die von Wasser oder Parfumöl verschieden sind. Die Zusatzstoffe sind in vielen Bereichen unerwünscht, da sie die natürliche Reinheit des Systems mindern, was beispielsweise eine Änderung des Dufteindrucks der Parfumölemulsion bewirken oder sogar zu Unverträglichkeitsreaktionen bei Menschen mit hohem Allergisierungspotential führen kann.

Es bestand also ein Bedarf an konzentrierten Parfumölemulsionen, die in reinerer Form vorliegen.

Gelöst wurde dieser Aufgabe durch die Bereitstellung eines Duftstoff- bzw. Parfumölkonzentrats in Form wässriger Emulsionen, enthaltend wenigstens 30 Gew.-% Parfumöl(e), wobei der Gehalt der beiden Komponenten Wasser und Parfumöl(e) einen Wert von zusammen 96 Gew.-%, vorzugsweise 97 Gew.-%, vorteilhafterweise 98 Gew.-%, in sehr vorteilhafter Weise 99 Gew.-%, insbesondere aber 99,5 Gew.-% übersteigt, bezogen auf das gesamte Konzentrat.

Die resultierende Emulsion ist vorteilhafterweise nicht brennbar oder entflammbar, kann folglich problemlos gehandhabt und weiterverarbeitet werden. Dies ist sehr vorteilhaft, da die originären Parfumöle in der Regel einen ausgesprochen niedrigen Flammpunkt aufweisen und deshalb Probleme bei der Lagerung, Verarbeitung und Handhabung bereiten. So werden Parfumöle in der Regel nur in geringen Mengen bevorratet und müssen von besonders geschultem Personal gehandhabt werden. Einige Parfumöle können infolge ihres niedrigen Flammpunktes gar nicht eingesetzt oder industriell gehandhabt werden, ohne großen Aufwand zu betreiben. Die Handhabung des erfindungsgemäßen Mittels läuft dagegen völlig unproblematisch und ohne größere Geruchsbelästigung ab.

In einer bevorzugten Ausführungsform weisen die Emulsionen mittlere Tröpfchengrößen (d₅₀) auf, die im wesentlichen größer als 0,1 µm sind und eine Obergrenze von 5 µm im wesentlichen nicht überschreiten. Es hat sich herausgestellt, daß insbesondere ein solcher Tröpfchengrößenbereich zu besonders stabilen Parfumölkonzentraten bzw. -emulsionen führt. Die mittlere Tröpfchengrößen (d₅₀) ist der Merkmalswert, bei dem die Verteilungssumme der Tröpfchendurchmesser den Wert 0,5 = 50% annimmt. Z. B: bedeutet die Angabe eines d₅₀ von a µm, daß von dem betrachteten Gut 50(Massen-)% der Tröpfchen einen Durchmesser größer als a µm und 50(Massen-)% einen kleineren Durchmesser als a µm aufweisen.

Es kann aber für manche Anwendungen, insbesondere im kosmetischen Bereich, auch bevorzugt sein, wenn die Emulsionen geringere mittlere Tröpfchengrößen (d₅₀) aufweisen und als Nanoemulsion vorliegen. Dementsprechend ist nach einer anderen bevorzugten Ausführungsform die Tröpfchengröße d₅₀ der Emulsion nicht größer als 400 nm, vorzugsweise nicht größer als 300 nm, vorteilhafterweise nicht größer als 250 nm, in weiter vorteilhafter Weise nicht größer als 200 nm, in noch vorteilhafterer Weise nicht größer als 150 nm, insbesondere wird ein Wert von 100 nm nicht überstiegen. Erfindungsgemäße Mikroemulsionen mit einer Tröpfchengröße d₅₀ nicht kleiner als 10 nm, vorzugsweise nicht kleiner als 25 nm, vorteilhafterweise nicht kleiner als 40 nm, insbesondere einen Wert von 60 nm nicht unterschreitend, sind dabei ganz besonders bevorzugt und stellen eine besonders vorteilhafte Ausführungsform der Erfindung dar.

Nanoemulsionen und deren Herstellung wurden bereits in der Patentliteratur beschrieben. Eine Übersicht zu Herstellung und Anwendung von Nano- und Mikroemulsionen wird gegeben durch H.Eicke im SÖFW-Journal, 118, 311 (1992) und Th.Förster et al. im SÖFW-Journal, 122, 746 (1996).

Gemäß einer bevorzugten Ausführungsform enthalten die Mittel mindestens 40 Gew.-%, vorzugsweise mindestens 50 Gew.-%, vorteilhafterweise mindestens 53 Gew.-%, insbesondere mindestens 55 Gew.-%, in besonders vorteilhafter mindestens 60 Gew.-% an Parfumöl(en).

Überraschenderweise wurde gefunden, daß Mittel mit solchen Parfumölanteilen ebenfalls besonders stabile Parfümökonzentrate bzw. -emulsionen darstellen.

Der Parfumölanteil sollte aus Stabilitätsgründen vorzugsweise aber auch nicht zu hoch angesetzt werden, so daß gemäß einer bevorzugten Ausführungsform das Mittel nicht mehr als 90 Gew.-% an Parfumöl(en) enthält.

Als Parfumöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 - 18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Die Parfumöle bzw. Duftstoffe können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfumöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Der Wasseranteil des Parfumölkonzentrates sollte aus Stabilitätsgründen vorzugsweise ebenfalls nicht zu hoch angesetzt werden, so daß gemäß einer bevorzugten Ausführungsform das Mittel weniger als 60 Gew.-%, vorteilhafterweise weniger als 50 Gew.-%, insbesondere weniger als 40 Gew.-% an Wasser enthält.

Die erfindungsgemäßen Mittel enthalten vorzugsweise auch einen Emulgator bzw. Emulgatoren.

Der Emulgator ist gemäß einer bevorzugten Ausführungsform der Erfindung ausgewählt aus der Gruppe der nichtionischen, zwitterionischen, ampholytischen, kationischen und/oder anionischen Emulgatoren.

Geeignete Emulgatoren sind beispielsweise die im "International Cosmetic Ingredient Dictionary and Handbook", 7.Auflage, Band 2 im Abschnitt 'Surfactants', insbesondere im Unterabschnitt 'Surfactants-Emulsifying Agents' aufgeführten Emulgatoren. Mit dem Begriff des Emulgators ist hier die Gesamtheit der grenzflächenaktiven Hilfsmittel zur Herstellung und Stabilisierung von Emulsionen gemeint, so daß also im Rahmen dieser Erfindung der Begriff des "Co-Emulgators" durch den Oberbegriff Emulgator mitumfasst ist. Die in anderen Schriften mitunter als "Co-Emulgatoren" bezeichneten Emulgatoren zeichnen sich üblicherweise durch ein Übergewicht des hydrophoben Molekülteils aus. Sie sind daher in der Regel etwas weniger wasserlöslich, können zur Ausbildung von Gelen und lamellaren Flüssigkristallen neigen und so die Viskosität einer Emulsion erhöhen.

Als zwitterionische Emulgatoren werden vorzugsweise solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Emulgatoren sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung "*Cocamidopropyl Betaine*" bekannte Fettsäureamid-Derivat.

Unter ampholytischen Emulgatoren werden solche oberflächenaktiven Verbindungen verstanden, die außer einer C8/18-Alkyl- oder -Acylgruppe im Molekül mindestens eine freie Aminogruppe und mindestens eine -COOH- oder -SO3H- Gruppe enthalten und zur Ausbildung innerer Salze befähigt sind. Beispiele für geeignete ampholytische Emulgatoren sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Emulgatoren sind das N-Kokosalkylaminopropionat, das Kokosacylaminoethylaminopropionat und das C12/18-Acylsarcosin.

Nichtionische Emulgatoren sind vorzugsweise ausgewählt aus mindestens einer der folgenden Substanzklassen:
- alkoxylierte Fettsäurealkylester der Formel

   R¹CO-(OCH₂CHR²)ₓOR³,

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäurealkanolamide und Fettamine,
- Fettsäure-N-alkylglucamide,
- C₈-C₂₂-Alkylamin-N-oxide,
- Alkylpolygykoside entsprechend der allgemeinen Formel RO-(Z)ₓ wobei R für eine C₈-C₁₆-Alkylgruppe, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht.
   Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zuckereinheiten. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weitere erfindungsgemäß geeignete nichtionische Emulgatoren sind vorzugsweise ausgewählt aus den Anlagerungsprodukten von 4 bis 100 Ethylenoxid-Einheiten an gegebenenfalls gehärtete Mono-, Di- und Triglyceride von C₈₋₂₂-Fettsäuren, den Anlagerungsprodukten von 5 bis 40 Ethylenoxid-Einheiten an C₈₋₂₂-Fettalkohole, was besonders bevorzugt ist, sowie den Anlagerungsprodukten von 2 bis 50 Ethylenoxid-Einheiten und 2 bis 35 Propylenoxid-Einheiten an C₃-C₅-Alkanole. Beispiele für ethoxylierte Mono-, Di- und Triglyceride von C₈₋₂₂-Fettsäuren mit 4 bis 60 Ethylenoxid-Einheiten sind hydriertes ethoxyliertes Castoröl (INCI-Bezeichnung z.B. PEG-40 Hydrogenated Castor Oil), Olivenölethoxylat (INCI-Bezeichnung: PEG-10 Olive Glycerides), Mandelölethoxylat, Nerzölethoxylat, Polyoxyethylenglycolcapryl-/caprinsäureglyceride, Polyoxyethylenglycerinmonolaurat und Polyoxyethylenglycolkokosfettsäureglyceride. Beispiele für geeignete ethoxylierte C₈₋₂₂-Fettalkohole sind Laureth-12, Laureth-23, Trideceth-8, Ceteareth-12, Ceteareth-15, Ceteareth-20, Ceteareth-30, Steareth-10, Steareth-15, Steareth-20, Steareth-30, Steareth-40, Oleth-10 oder Oleth-20. Beispiele für geeignete Polyethylenglycol-Polypropylenglycol-Mischether von C₃-C₅-Alkanolen sind die PEG-PPG-Addukte von 1-Propanol, 2-Propanol und iso-Propanol, 1-Butanol, 2-Butanol, iso-Butanol und 1-Pentanol, 2-Pentanol und Amylalkohol mit 2 - 50, bevorzugt 4 - 40 Ethylenoxid-Einheiten und 2 - 35, bevorzugt 4 - 30 Propylenoxid-Einheiten, insbesondere PPG-28-Buteth-35, PPG-26-Buteth-26, PPG-5-Buteth-5, PPG-25-Buteth-25, PPG-5-Buteth-20, PPG-33-Buteth-45, PPG-20-Buteth-30 oder PPG-12-Buteth-16.

Ebenfalls bevorzugte nichtionische Emulgatoren sind weiterhin die Ethylenoxid-Addukte von linearen C₃-C₂₂-Alkoholen mit einer mittleren Anzahl der Ethylenoxid-Einheiten von 1-30. Bevorzugt geeignete nichtionische Emulgatoren sind Ethylenoxid-Addukte von verzweigten C₃-C₂₈-Alkoholen, insbesondere von sogenannten Guerbet-Alkoholen, mit einer mittleren Anzahl von Ethylenoxid-Einheiten von 1-30.

Weitere bevorzugt geeignete nichtionische Emulgatoren sind Ethylenoxd-Propylenoxid-Mischaddukte von linearen C₃₋C₂₂-Alkoholen mit einer mittleren Anzahl an Ethylenoxid-Einheiten von 2-50, bevorzugt 4-40 und einer mittleren Anzahl an Propylenoxid-Einheiten von 2-35, bevorzugt 4-30.

Besonders bevorzugte nichtionische Emulgatoren sind Propylenoxid-Addukte von linearen C₃-C₂₂-Alkoholen. Die mittlere Anzahl der Propylenoxid-Einheiten beträgt 1-30, bevorzugt 5-25 und besonders bevorzugt 8-15. Geeignete propoxylierte Emulgatoren sind z. B. PPG-3-Myristylether (Witconol^{®} APM), PPG-14-Butylether (Ucon Fluid^{®} AP), PPG-15-Stearylether (Arlamol^{®} E), PPG-9-Butylether (Breox^{®} B25) und PPG-10-Butandiol (Macol^{®} 57), wobei PPG-14-Butylether und PPG-15-Stearylether besonders bevorzugt sind.

Bevorzugt können die emulgierten Parfumöle mindestens einen nichtionischen Emulgator mit einem HLB-Wert von 3 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionische O/W-Emulgatoren mit einem HLB-Wert von 10-15 sowie nichtionische W/O-Emulgatoren mit einem HLB-Wert von 3-6 können erfindungsgemäß besonders bevorzugt sein.

In einer besonders bevorzugten Ausführungsform sind ausschließlich nichtionische Emulgatoren enthalten, vorzugsweise nur ein einziger nichtionischer Emulgator, vorteilhafterweise ausgewählt aus den Anlagerungsprodukten von 5 bis 40 Ethylenoxid-Einheiten an C₈₋₂₂-Fettalkohole, insbesondere Eumulgin® B3 (Cetylstearylalkohol+30-EO; erhältlich über Cognis Deutschland GmbH). Ebenfalls höchst bevorzugte nichtionische Emulgatoren im Rahmen dieser besonders bevorzugten Ausführungsform sind die ethoxylierten Fettsäurealkanolamide, vorzugsweise ethoxylierte Kokosfettsäuremonoethanolamide, insbesondere Kokosfettsäuremonoethanolamide plus 4 Ethylenoxid -Einheiten, was beispielsweise dem kommerziellen Produkt Eumulgin® C4 entspricht (erhältlich über Cognis Deutschland GmbH). Bei Einsatz von Emulgatoren entsprechend dieser bevorzugten Ausführungsformen, insbesondere bei Einsatz von Eumulgin® B3 und/oder Eumulgin® C4, lassen sich erfindungsgemäße Parfumölkonzentrate mit herausragender Stabilität bereitstellen.

Nichtschäumende Emulgatoren sind außerordentlich bevorzugt.

Erfindungsgemäß geeignet sind ebenfalls kationische Emulgatoren, vorzugsweise vom Typ der quartären Ammoniumverbindungen, der Esterquats und der Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Emulgatoren weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Der große Vorteil der kationischen Emulgatoren besteht darin, daß sie den EmulsionsTröpfchen eine positive Ladung verleihen und auf diese Weise eine verstärkte Adsorption solcher Parfumöle aus der Emulsionsphase an negativ geladene Oberflächen, z.B. auf Textilfasern, bewirken.

Anionische Emulgatoren weisen vorzugsweise eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen auf. Zusätzlich können im Molekül Glycol- oder Polyglycolether-Gruppen, Ester-, Ether- und Amidgruppen sowie Hydroxylgruppen enthalten sein. Beispiele für geeignete anionische Emulgatoren sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Acylglutamate der Formel (I), 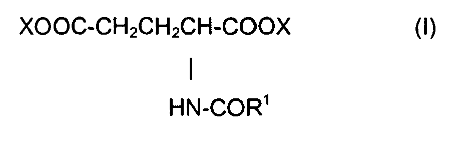 in der R¹CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (II), 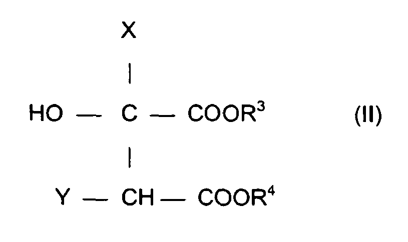 in der X=H oder eine -CH₂COOR²-Gruppe ist, Y=H oder -OH ist unter der Bedingung, dass Y=H ist, wenn X=-CH₂COOR² ist, R², R³ und R⁴ unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, daß wenigstens eine der Gruppen R², R³ oder R⁴ ein Rest Z ist,
- Ester der Sulfobernsteinsäure oder der Sulfosuccinate der allgemeinen Formel (III), 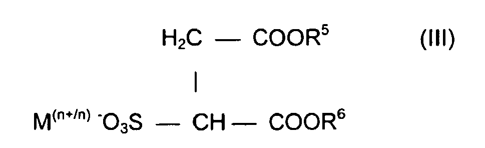 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R⁵ und R⁶ unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R⁵ oder R⁶ ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1-12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.

Bevorzugte anionische Emulgatoren sind Acylglutamate, Acylisethionate, Acylsarcosinate und Acyltaurate, jeweils mit einem linearen oder verzweigten Acylrest mit 6 bis 22 Kohlenstoffatomen und 0, 1, 2 oder 3 Doppelbindungen, der in besonders bevorzugten Ausführungsformen aus einem Octanoyl-, Decanoyl-, Lauroyl-, Myristoyl-, Palmitoyl- und Stearoylrest ausgewählt ist, Ester der Weinsäure, Zitronensäure oder Bernsteinsäure bzw. der Salze dieser Säuren mit alkylierter Glucose, insbesondere die Produkte mit der INCI-Bezeichnung Disodium Coco-Glucoside Citrate, Sodium Coco-Glucoside Tartrate und Disodium Coco-Glucoside Sulfosuccinate, Alkylpolyglycolethersulfate und Ethercarbonsäuren mit 8 bis 18 C-Atomen in der Alkylgruppe und bis zu 12 Ethoxygruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Ethoxygruppen.

Der Anteil der Emulgatoren am gesamten Mittel ist infolge des großen Anteils der Komponenten Wasser und Parfumöl(e) recht gering; er beträgt gemäß einer bevorzugten Ausführungsform jedoch mindestens 0,1 Gew.-%, aber weniger als 4 Gew.-%, vorzugsweise weniger als 2,5 Gew.-%, vorteilhafterweise weniger als 2,0 Gew.-%, in sehr vorteilhafter Weise weniger als 1,5 Gew.-%, in überaus vorteilhafter Weise weniger als 1,0 Gew.-%, insbesondere aber weniger als 0,7 Gew.-%, bezogen auf das gesamte Mittel.

Gemäß einer bevorzugten Ausführungsform wird eine Mindestmenge an Emulgator von 0,12 Gew.-%, vorzugsweise von 0,175 Gew.-%, vorteilhafterweise von 0,2 Gew.-% nicht unterschritten.

Es ist besonders vorteilhaft, mit derart geringen Mengen an Emulgator auszukommen, da das emulgierte Parfumöl auf diese Weise auch problemlos für solche Anwendungen weiterverwendet werden kann, bei dem Emulgatoren unter Umständen nur in sehr geringen Mengen erwünscht sind, beispielsweise bei manchen kosmetischen Anwendungszwecken, z. B. solchen, die sich mit irritierter Haut befassen.

Die vorgenannten Ausführungen bezüglich der Emulgatoren treffen auch auf die erfindungsgemäßen Nanoemulsionen zu. Allerdings gibt es hier weitere vorteilhafte Ausführungsformen, die zu einer weiteren Erhöhung der Stabilität der Emulsion führen. Vorteilhaft ist es hier, wenn eine erfindungsgemäße Nanoemulsion zumindest zwei Emulgatoren aufweist. Gemäß einer bevorzugten Ausführungsform umfaßt eine erfindungsgemäße Nanoemulsion ein Emulgatorsystem aus wenigstens einem lipophilen, vorzugsweise lipophilen kationischen, Emulgator, sowie wenigstens einem hydrophilen, vorzugsweise hydrophilen nichtionischen, Emulgator.
Wenn im Falle der erfindungsgemäßen Nanoemulsionen gleichzeitig ein kationischer und ein nichtionischer Emulgator enthalten ist, so liegt das Mengenverhältnis kationischer zu nichtionischem Emulgator vorteilhafterweise im Bereich von 70:1 bis 1:3, insbesondere von 30:1 bis 1:2, vorzugsweise von 10:1 bis 1:1, und besonders bevorzugt von 5:1 bis 2:1.

Als lipophil gelten Emulgatoren im Sinne dieser Anmeldung im wesentlichen dann, wenn sie zum einen HLB-Werte kleiner/gleich 8 aufweisen und wenn sie zum anderen vorteilhafterweise in C₁₂-C₂₀ Triglyceriden überwiegend löslich bzw. mit diesen mischbar sind. Lipophilie kann sich u. a. beispielsweise dann ergeben, wenn die Emulgatoren etwa Kohlenwasserstoffreste mit 6 bis 22 Kohlenstoffatomen aufweisen oder etwa Arylreste enthalten, um anschauliche, aber nicht einschränkende Beispiele zu geben. Lipophile Emulgatoren haben im wesentlichen einen wenig polaren, eher apolaren Charakter. Bevorzugte lipophile Emulgatoren im Sinne dieser Erfindung stellen lipohile, ethoxylierte Fettalkoholen (C₁₂-C₂₀-Fettalkohole mit 1 bis 3 EO-Einheiten) dar. Geeignet sind auch Ethylenoxid/Propylenoxid-modifizierte Silikonölemulgatoren.

Demgegenüber gelten Emulgatoren im Sinne dieser Anmeldung im wesentlichen dann als hydrophil, wenn sie zum einen einen HLB-Wert von größer/gleich 13 aufweisen und wenn sie zum anderen vorteilhafterweise in Wasser überwiegend löslich sind bzw. mit diesem mischbar. Hydrophile Emulgatoren haben im wesentlichen einen polaren Charakter. Hydrophilie kann sich u. a. beispielsweise dann ergeben, wenn der Emulgator etwa HydroxyGruppe(n), Ester-Gruppe(n), Ether-Gruppe(n) oder Glycerin-Gruppe(n) enthält, um anschauliche, aber nicht einschränkende Beispiele zu geben.

Der Begriff des HLB-Wertes ist dem Fachmann bekannt. Der HLB-Wert ist ein von Griffin (1950) eingeführtes Maß für die Wasser- bzw. Öl-Löslichkeit von Tensiden bzw. Emulgatoren und die Stabilität von Emulsionen. Experimentell läßt sich der HLB-Wert z. B. durch die Phenol-Titrationsmethode bestimmen, indem man die Tensid- bzw. Emulgator-Lösung mit 5%-iger Phenol-Lösung bis zur Trübe versetzt. Ferner kann der HLB-Wert (gas-)-)chromatographisch, durch Bestimmung der Dielektrizitätskonstante oder kolorimetrisch ermittelt werden. Ausführliche Informationen hierzu und auch Listen der HLB-Werte von Handelsemulgatoren finden sich in der einschlägigen Fachliteratur oder in Nachschlagewerken wie z. B. Kirk-Othmer Encyclopedia of Chemical Technology by John Wiley & Sons. Die HLB-Skala reicht in der Regel von 1 bis 20. Substanzen mit niedrigem HLB-Wert (3 bis 8) gelten gemeinhin als lipohil und gelten im allgemeinen als gute W/O-Emulgatoren, während Substanzen mit höherem HLB-Wert (8 bis 18) als hydrophil gelten und gemeinhin als O/W-Emulgatoren wirken.

Besonders vorteilhaft ist es, wenn der in der Nanoemulsion enthaltene nichtionische, hydrophile Emulgator ausgewählt ist aus ethoxylierten Fettalkoholen und/oder ethoxylierten Fettsäurealkanolamiden. Besonders bevorzugt sind dabei mit Blick auf die ethoxylierten Fettalkoholen die Anlagerungsprodukte von 5 bis 40 Ethylenoxid-Einheiten an C₈₋₂₂-Fettalkohole, wobei insbesondere Eumulgin® B3 (Cetylstearylalkohol+30-EO; erhältlich über Cognis Deutschland GmbH) überaus bevorzugt ist. Besonders bevorzugt mit Blick auf die ethoxylierten Fettsäurealkanolamide sind vorzugsweise die ethoxylierten Kokosfettsäuremonoethanolamide, insbesondere Kokosfettsäuremonoethanolamide plus 4 Ethylenoxid -Einheiten, was beispielsweise dem kommerziellen Produkt Eumulgin® C4 entspricht (erhältlich über Cognis Deutschland GmbH).

Ebenfalls besonders vorteilhaft ist es, wenn es sich bei den in der Nanoemulsion enthaltenen kationischen Emulgatoren um quartäre Ammoniumverbindungen handelt, vorteil-, hafterweise um alkylierte quartäre Ammoniumverbindungen, vorzugsweise mit ein, zwei oder drei hydrophoben Gruppen, die insbesondere über Ester- oder Amidobindungen mit einem quaternierten Di- bzw. Triethanolamin oder einer analogen Verbindung verknüpft sind. Beispielsweise ist N-Methyl-N(2-hydroxyethyl)-N,N-(ditalgacyloxyethyl)ammoniummethosulfat oder um N-Methyl-N(2-hy-droxyethyl)-N,N-(dipalmitoylethyl)ammoniummethosulfat sehr vorteilhaft.

Auch für die Nanoemulsion gilt erfindungsgemäß, daß der Anteil der Emulgatoren am gesamten Mittel recht gering ist. Gemäß einer bevorzugten Ausführungsform enthält eine erfindungsgemäße Nanoemulsion nicht mehr als 3,5 Gew.-%, vorzugsweise nicht mehr als 3 Gew.-%, vorteilhafterweise nicht mehr als 2,5 Gew.-%, insbesondere nicht mehr als 2 Gew.-%, mindestens jedoch 0,1 Gew.-% an liphophilen Emulgatoren. Ebenso enthält eine erfindungsgemäße Nanoemulsion gemäß einer bevorzugten Ausführungsform nicht mehr als 3,5 Gew.-%, vorzugsweise nicht mehr als 3 Gew.-%, vorteilhafterweise nicht mehr als 2,5 Gew.-%, insbesondere nicht mehr als 2 Gew.-%, mindestens jedoch 0,1 Gew.-% an hydrophilen Emulgatoren.

Vorzugsweise enthält das erfindungsgemäße Mittel auch Verdickungsmittel. Der Anteil der Verdickungsmittel am gesamten Mittel ist infolge des großen Anteils der Komponenten Wasser und Parfumöl(e) ebenfalls recht gering, er beträgt gemäß einer bevorzugten Ausführungsform jedoch mindestens 0,1 Gew.-%, aber weniger als 4 Gew.-%, vorzugsweise weniger als 2,5 Gew.-%, vorteilhafterweise weniger als 1,9 Gew.-%, in sehr vorteilhafter Weise weniger als 1,5 Gew.-%, in überaus vorteilhafter Weise weniger als 1,0 Gew.-%, insbesondere aber weniger als 0,7 Gew.-%, bezogen auf das gesamte Mittel.

Gemäß einer bevorzugten Ausführungsform sind geeignete Verdickungsmittel ausgewählt aus der Gruppe der
a) Polysaccharide, insbesondere Xanthan-Gum, Guar-Derivate, Gummi arabicum, Karaya-Gummi, Traganth, Taragummi, Gellan, Carrageen, Johannisbrotkernmehl, Agar-Agar, Alginate, Pektine und/oder Dextrane,
b) organische vollsynthetische Verdickungsmittel, insbesondere Polyacrylate, Polyacrylamide, Polyvinylpyrrolidon, Polyvinylalkohol, Polyethylenglykole, hydrophob modifizierte Polyether, Polyurethane, Styrol-Maleinsäureanhydrid-Copolymerisate, deren Salze und/oder deren Derivate,
c) Cellulose-Derivate, insbesondere Hydroxyethylcellulose, Carboxymethylcellulose, Hydroxypropylmethylcellulose, Hydroxypropylcellulose, Ethylhydroxyethylcellulose, Methylcellulose,
d) Stärke-Fraktionen und Derivate, insbesondere Amylose, Amylopektin und Dextrine,
e) Tone, insbesondere Bentonit.

Im Falle der erfindungsgemäßen Nanoemulsionen ist es weiterhin vorteilhaft, wenn mindestens 0,05 Gew.-%, vorzugsweise zumindest 0,1 Gew.-%, vorteilhafterweise zumindest 0,15 Gew.-%, insbesondere zumindest 0,2 Gew.-%, jedoch nicht mehr als 3 Gew.-%, vorzugsweise nicht mehr als 2,5 Gew.-%, vorteilhafterweise nicht mehr als 2,0 Gew.-%, in sehr vorteilhafter Weise nicht mehr als 1,5 Gew.-%, in noch vorteilhafterer Weise nicht mehr als 1,0 Gew.-%, in überaus vorteilhafter Weise nicht mehr als 0,75 Gew.-%, in vorteilhaftester Weise nicht mehr als 0,5 Gew.-% an Verdickungsmitteln enthalten ist.

Im Gegensatz zu den normalen Emulsionen sind die Nanoemulsionen aufgrund der Tröpfchenfeinheit vorteilhafterweise so stabil, das ein Verdickungsmittel vorzugsweise nicht notwendig ist. In einer bevorzugten Anwendungsform betreffend Emulsionen mit einem Tröpfchendurchmesser d₅₀ < 200 nm wird auf den Zusatz von Verdickungsmitteln verzichtet.

Gemäß einer bevorzugten Ausführungsform wird für die normale Emulsion eine Mindestmenge an Verdickungsmittel im Mittel von 0,12 Gew.-%, vorzugsweise von 0,2 Gew.-%, bezogen auf das Mittel, nicht unterschritten.

Auf die Mitwirkung niederer Alkohole kann im Rahmen der Erfindung vorzugsweise sehr weitgehend verzichtet werden. Die erfindungsgemäßen Konzentrate sind daher vorzugsweise von niederen Alkoholen im wesentlichen frei. Soweit kleinere Mengen z. B. durch die Parfumöle selbst oder durch andere Rohstoffe, z. B. durch die kationischen Emulgatoren, in die Konzentrate gelangen, sollte der Anteil solcher Alkohole mit 1 - 4 C-Atomen im Konzentrat vorzugsweise weniger als 1 Gew.-% betragen.

Die erfindungsgemäßen Mittel haben den Vorteil, daß sie Parfumöle in sehr hoher Konzentration, vorzugsweise in Konzentrationen bis zu 90 Gew.-% an Parfumöl, bezogen auf das gesamte Mittel, enthalten können. Ein Nutzen der hohen Parfumölkonzentration liegt beispielsweise darin, daß die emulgierten Parfumöle im Vergleich zu den in der Handhabung sehr unvorteilhaften originären Parfumölen nur unwesentlich mehr Lagerraum bei deutlich reduziertem Sicherheitsaufwand benötigen.

Die zum Zeitpunkt der Anmeldung beste Konfiguration der Erfindung für normale Emulsionen besteht nach Ansicht der Anmelderin in solchen erfindungsgemäßen Mitteln, welche neben den beiden grundsätzlichen Komponenten Parfumöl und Wasser zumindest einen nichtionischen Emulgator enthalten, vorzugsweise nur einen einzigen nichtionischen Emulgator, welcher vorteilhafterweise ausgewählt ist aus den Anlagerungsprodukten von 5 bis 40 Ethylenoxid-Einheiten an C₈₋₂₂-Fettalkohole, insbesondere Eumulgin® B3 (Cetylstearylalkohol+30-EO; erhältlich über Cognis Deutschland GmbH) und/oder den ethoxylierten Fettsäurealkanolamiden, vorzugsweise ethoxylierte Kokosfettsäuremonoethanolamide, insbesondere Kokosfettsäuremonoethanolamide plus 4 Ethylenoxid-Einheiten, was beispielsweise dem kommerziellen Produkt Eumulgin® C4 entspricht (erhältlich über Cognis Deutschland GmbH). In dieser besten Konfiguration ist ebenfalls zumindest ein Verdickungsmittel enthalten, vorzugsweise nur ein einziges Verdickungsmittel, vorteilhafterweise ausgewählt aus der Gruppe der Polysaccharide, hierunter insbesondere ausgewählt aus Xanthan-Gum, Guar-Derivate, Gummi ara-bicum, Karaya-Gummi, Traganth, Taragummi, Gellan, Carrageen, Johannisbrotkernmehl, Agar-Agar, Alginate, Pektine und/oder Dextrane, am vorteilhaftesten ist aber Xanthan-Gum, und /oder ausgewählt aus der Gruppe der Cellulose-Derivate, hierunter vorzugsweise ausgewählt aus Hydroxyethylcellulose, Carboxymethylcellulose, Hydroxypropyl-methylcellulose, Hydroxypropylcellulose, Ethylhydroxyethyl-cellulose, Methylcellulose, am vorteilhaftesten ist aber die Hydroxyethylcellulose. In dieser besten Konfiguration enthält das Parfümölkonzentrat keine anderen als die vorgenannten Emulgatoren und Verdickungsmittel. In dieser besten Konfiguration enthält das Parfumölkonzentrat vorteilhafterweise weniger als 2,0 Gew.-% jedoch vorzugsweise mindestens 0,1 Gew.-% jeweils an Verdicker als auch an Emulgator. In dieser besten Konfiguration enthält das Parfumölkonzentrat vorzugsweise zumindest 40 Gew.-% Parfumöl und vorteilhafterweise weniger als 60 Gew.-%, insbesondere weniger als 50 Gew.-% an Wasser.

Parfumölkonzentrate, welche die zuvor dargestellten Kriterien der besten Konfiguration erfüllen, zeichnen sich nicht zuletzt durch eine überragende Stabilität aus.

Es ist ein Erzeugnis möglich, welches ein erfindungsgemäßes Parfümölkonzentrat und zumindest einen Wirk-, Hilfs- und/oder Zusatzstoff enthält, insbesondere ausgewählt aus folgender Auflistung:
- haarkonditionierende Verbindungen wie Phospholipide, beispielsweise Sojalecithin, Ei-Lecitin und Kephaline, sowie Silikonöle,
- Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie
- quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien
- Geruchsverstärker
- Transferstoffe.

In einer bevorzugten Ausführungsform ist bzw. sind die enthaltenen Wirk-, Hilfs- und/oder Zusatzstoff(e) dabei in so geringen Konzentrationen enthalten, daß selbst in dem resultierenden Erzeugnis der Gehalt der beiden Komponenten Wasser und Parfumöl(e) einen Wert von zusammen 96 Gew.-% übersteigt, bezogen auf das gesamte Erzeugnis.

Gleichwohl kann es je nach gewünschtem Anwendungsgebiet des Erzeugnisses auch erwünscht sein, daß der oder die enthalten Wirk-, Hilfs- und/oder Zusatzstoff(e) in größeren Anteilen im resultierenden Erzeugnis enthalten sind, so daß solche Erzeugnisse ebenfalls eine bevorzugte Ausführungsform im Hinblick auf diese Erzeugnisse darstellen.

Bezüglich anderer und/oder weiterer fakultativer Komponenten sei darauf verwiesen, daß die Auswahl solcher Komponenten im Hinblick auf die Bereitstellung entsprechender Erzeugnisse im wesentlichen davon abhängt, welchem Verwendungszweck das resultierende Erzeugnis zugeführt werden soll. Soll das emulgierte Parfumöl beispielsweise im Rahmen des kosmetischen Anwendungsbereich weiter verarbeitet werden oder dort zum Einsatz gelangen, so sei, was die Natur sowie die Mengen der dabei sinnvoll einsetzbaren Komponenten anbelangt, ausdrücklich auf die dem Fachmann wohlbekannten einschlägigen Handbücher, z. B. die Monographie von K. H. Schrader, Grundlagen und Rezepturen der Kosmetika, 2. Auflage, Hüthig Buch Verlag Heidelberg, 1989, verwiesen. In Analogie hierzu sind dem jeweiligen Fachmann andere einschlägige Handbücher zu anderen Verwendungszwecken bzw. Einsatzgebieten wohlvertraut.

Bevorzugt ist es, wenn im wesentlichen wasserlösliche Wirk-, Hilfs- und Zusatzstoffe in das bereits fertig emulgierte Parfumölkonzentrat eingearbeitet, beispielsweise eingerührt werden, so daß entsprechende Erzeugnisse erhalten werden.

Ein weiterer Gegenstand der Erfindung ist ein Verfahren zur Herstellung eines Mittels, das die zuvor beschriebenen Merkmale aufweist. Dieses Verfahren, welches ein Zwei-Topf-Verfahren ist, umfasst die Schritte:
(a) Zugabe von Verdickungsmittel zu Wasser bei Rührung des Gemisches in einem ersten Gefäß,
(b) Zugabe von Emulgator zum Parfumöl unter Rührung in einem zweiten Gefäß
(c) Zugabe des Inhalts des zweiten Gefäßes in das Gemisch des ersten Gefäßes unter Einsatz einer Homogenisierungsmaschine.

Gemäß einer bevorzugten Ausführungsform des Verfahrens erfolgt die Zugabe des Emulgators zum Parfumöl bei Temperaturen unterhalb 60°C, vorteilhafterweise im Temperaturbereich von 25 bis 55°C.

Gemäß einer weiteren bevorzugten Ausführungsform des Verfahrens wird das Gemisch des ersten Gefäßes vor der Parfumöl/Emulgator-Zugabe auf eine Temperatur unterhalb 50°C, vorzugsweise unterhalb 40°C, vorteilhafterweise auf eine Temperatur im Bereich von 20-35°C temperiert.

Ein weiterer Gegenstand der Erfindung ist ein Verfahren, das in einem einzigen Gefäß durchgeführt wird, wobei zuerst das Wasser vorgelegt wird, danach das Verdickungsmittel und der Emulgator, vorteilhafterweise gleichzeitig, unter Rührung zugegeben werden, und anschließend das Parfumöl unter Einsatz einer Homogenisiermaschine zugegeben wird. Der Vorteil dieses Verfahrens liegt in der vorteilhafteren Prozessökonomie, da es sich um ein Ein-Topf-Verfahren handelt. Gemäß einer bevorzugten Ausführungsform zeichnet sich dieses Verfahren dadurch aus, daß die Zugabe des Verdickungsmittels bei Temperaturen unterhalb 60°C, vorzugsweise unterhalb 50°C, vorteilhafterweise bei Temperaturen im Bereich von 15-30°C erfolgt, und daß die Zugabe des Emulgators während oder nach der Temperierung des Gemisches auf eine Temperatur unterhalb 70°C, vorzugsweise unterhalb 60°C, vorteilhafterweise bei Temperaturen im Bereich 35-55°C erfolgt und daß das Gemisch vor der Parfumölzugabe auf eine Temperatur unterhalb 50°C, vorzugsweise unterhalb 40°C, vorteilhafterweise auf eine Temperatur im Bereich von 20-35°C abgekühlt wird.

Trotz der Vorteilhaftigkeit dieses Verfahrens, besteht die beste Verfahrenskonfiguration in dem Zwei-Topf-Verfahren.

Gemäß einer weiteren bevorzugten Ausführungsform erfolgt das erfindungsgemäße Verfahren unter Gaszugabe, wobei das Gas vorzugsweise ausgewählt ist aus der Gruppe der Edelgase, Stickstoff und/oder Kohlendioxid. Die Gaszugabe erfolgt bevorzugt nach dem Mischungsprinzip, d. h. durch Einleitung des Gases in die flüssige Mischung. Vorteilhafterweise ist das Mischungsprinzip mit dem Druckwechselprinzip zu kombinieren, d. h. es wird eine, vorzugsweise wiederholte Evakuierung und Belüftung des Gemisches mit dem Gas durchgeführt.

Durch die Gaszugabe werden unerwünschte Oxidationsreaktionen vermieden und die Lagerstabilität des Mittels verbessert.

Die Gaszugabe erfolgt vorteilhafterweise nach Fertigstellung des Parfumölkonzentrates in das Parfumölkonzentrat. Bei besonders empfindlichen Rezepturen bzw. Parfumölen ist es ebenso vorteilhaft während der gesamten Dauer der Zubereitung eine Gaszugabe zu gewährleisten. Bei dem Verfahren mit den 2 Gefäßen werden dabei vorteilhafterweise beide Gefäße bzw. die darin enthaltenen Mischungen mit Gas versorgt.

Gemäß einer weiteren bevorzugten Ausführungsform können im Anschluß an das Verfahren zur Herstellung des Parfumölkonzentrates in das fertige Konzentrat Feststoffe, vorzugsweise feine Pulver, einemulgiert werden, so daß entsprechende Erzeugnisse resultieren. Insbesondere handelt es sich bei diesen Feststoffen um waschmittelübliche Zusatzstoffe, welche vorteilhafterweise aus der Gruppe der Zeolithe, Bentonite, Silikate, Phosphate, Harnstoff und/oder dessen Derivate, Sulfate, Carbonate, Citrate, Citronensäure, Acetate und/oder Salze der Aniontenside ausgewählt sind. Feine Pulver bedeutet, daß die Feststoffe vorzugsweise einen d₅₀-Wert von weniger als 0,2 mm, vorteilhafterweise weniger als 0,1 mm, insbesondere weniger als 0,05 mm aufweisen.

Möglich ist dementsprechend ein Erzeugnis, enthaltend ein erfindungsgemäßes Parfümölkonzentrat und Feststoffe, vorzugsweise waschmittelübliche Feststoffe, vorteilhafterweise ausgewählt aus der Gruppe der Zeolithe, Bentonite, Silikate, Phosphate, Harnstoff und/oder dessen Derivate, Sulfate, Carbonate, Citrate, Citronensäure, Acetate und/oder Salze der Aniontenside, insbesondere in Form feiner Pulver. Feine Pulver bedeutet, daß die Feststoffe vorzugsweise einen d₅₀-Wert von weniger als 0,2 mm, vorteilhafterweise weniger als 0,1 mm, insbesondere weniger als 0,05 mm aufweisen.

Möglich ist ein Erzeugnis, welches ein erfindungsgemäßes Parfümölkonzentrat und einen lipophilen Verdicker enthält, vorzugsweise ausgewählt aus der Gruppe der Fettalkohole, Fettalkoholethoxylate und/oder deren Derivaten, Fettsäuren, Fettsäurealkanolamidethoxylaten, Paraffine und/oder Silikonöle, dabei ist der lipophile Verdicker vorteilhafterweise in Mengen von 0,05 bis 3 Gew.-%, insbesondere von 0,1 bis 1 Gew.-% enthalten, jeweils bezogen auf das Parfümölkonzentrat, wobei ein solches Erzeugnis sich in sehr vorteilhafter Weise dadurch auszeichnet, daß es eine retardierte Duftwirkung aufweist. Solche angedickten Mittel zeichnen sich dadurch aus, daß sie infolge der erhöhten Viskosität der Parfumöle eine wesentlich längere Duftwirkung bzw. Duftdauer aufweisen als nicht angedickte Parfumöle. Die Duftwirkung entfaltet sich hierbei kontinuierlich und zieht sich über einen signifikant verlängerten Zeitraum hin. Als lipophil gelten Verdicker im Sinne dieser Anmeldung im wesentlichen dann, wenn sie vorteilhafterweise in C₁₂-C₂₀ Triglyceriden überwiegend löslich bzw. mit diesen mischbar sind. Lipophilie kann sich u. a. beispielsweise dann ergeben, wenn die Verdicker etwa Kohlenwasserstoffreste mit 6 bis 22 Kohlenstoffatomen aufweisen oder etwa Arylreste enthalten, um anschauliche, aber nicht einschränkende Beispiele zu geben. Bevorzugte lipophile Verdicker im Sinne dieser Erfindung stellen die Silikonöle dar. Demgegenüber gelten Verdicker im Sinne dieser Anmeldung im wesentlichen dann als hydrophil, wenn sie vorteilhafterweise in Wasser überwiegend löslich sind bzw. mit diesem mischbar. Hydrophilie kann sich u. a. beispielsweise dann ergeben, wenn die Verdicker etwa Hydroxy-Gruppe(n), Ester-Gruppe(n), Ether-Gruppe(n) oder Glycerin-Gruppe(n) enthält, um anschauliche, aber nicht einschränkende Beispiele zu geben.

Demgemäß ist ein Verfahren zur Herstellung eines eben beschriebenen Erzeugnisses möglich, wobei man zu einem erfindungsgemäßen Parfumölkonzentrat, unter Homogenisierung einen lipophilen Verdicker vor oder nach der Emulgierung, vorzugsweise in Mengen von 0,05 bis 3 Gew.-%, insbesondere in Mengen von 0,1 bis 1 Gew.-%, bezogen auf das gesamte Mittel, zufügt.

Gemäß einer bevorzugten Ausführungsform dieses Verfahrens wird in einem ersten Gefäß der lipophile Verdicker vorzugsweise unter Rühren zum Parfumöl gegeben und danach bei leicht erhöhter Temperatur der Emulgator zugegeben und gelöst, vorzugsweise 0,1 bis 1 Gewichtsteile Emulgator bezogen auf das Parfumölkonzentrat bestehend aus Parfumölemulsion und lipophilem Verdicker, wie beispielsweise Octanol, Decanol, Dodecanol oder Siliconöle. Diese Lösung wird nach Abkühlen in ein zweites Gefäß, welches eine Mischung aus Wasser und hydrophilen Verdicker, wie beispielsweise Hydroxyethylcellulose, enthält, eingerührt und anschließend homogenisiert.
Der Vorteil der Zugabe des lipophilen Verdickers vor der Emulgierung besteht darin, daß man eine sehr einheitliche Zusammensetzung der einzelnen Tröpfchen erhält.

Die Verwendung eines lipophilen Verdickers zur Erzeugung einer Parfumölemulsion mit retardierter Duftwirkung ist möglich.

Die erfindungsgemäßen Parfumöl-Konzentrate lassen sich sowohl mit Wasser verdünnen als auch wässrigen Zubereitungen zusetzen, ohne daß es zur Koaleszenz der emulgier ten Parfumöle kommt. Dies ist ein bedeutender Vorteil der erfindungsgemäßen Mittel und eröffnet weitreichende anwendungstechnische Perspektiven und Einsatzmöglichkeiten.

Ein anwendungstechnisch besonders bedeutender Aspekt der Erfindung ist daher die Verwendung der erfindungsgemäßen Parfumölkonzentrate zur Beduftung wässriger Zubereitungen, beispielsweise in Form wässriger Lösungen oder wässriger Dispersionen, jedweder Art. Solche Zubereitungen können z. B. kosmetische Mittel, vorzugsweise kosmetische Reinigungsmittel, wie Schaum- und Duschbadformulierungen, flüssige Seifen, Shampoos oder andere wässrige Körperreinigungsmittel sein.

Möglich ist daher ein kosmetische Erzeugnis, enthaltend ein erfindungsgemäßes Parfumölkonzentrat und zumindest einem kosmetisch wirksamen Stoff, vorzugsweise ausgewählt aus der Gruppe der hautpflegenden Aktivstoffe.

In einer bevorzugten Ausführungsform enthält ein solches Erzeugnis
a) 0,01 bis 75 Gew.-% eines erfindungsgemäßen Mittels, sowie
b) wenigstens 0,01 Gew.-% zumindest eines kosmetisch wirksamen Stoffes, vorzugsweise ausgewählt aus der Gruppe der hautpflegenden Aktivstoffe,
wobei die Gew.-% Angabe jeweils auf das gesamte Erzeugnis bezogen ist.

Dabei sind unter dem Begriff der hautpflegenden Aktivstoffe alle solchen Aktivstoffe zu verstehen, die der Haut einen sensorischen und/oder kosmetischen Vorteil verleihen. Die hautpflegende Aktivstoffe sind bevorzugt ausgewählt aus den nachfolgenden Substanzen:
a) Wachse wie beispielsweise Carnauba, Spermaceti, Bienenwachs, Lanolin und/oder Derivate derselben und andere.
b) Hydrophobe Pflanzenextrakte
c) Kohlenwasserstoffe wie beispielsweise Squalene und/oder Squalane
d) Höhere Fettsäuren, vorzugsweise solche mit wenigstens 12 Kohlenstoffatomen, beispielsweise Laurinsäure, Stearinsäure, Behensäure, Myristinsäure, Palmitinsäure, Ölsäure, Linolsäure, Linolensäure, Isostearinsäure und/oder mehrfach ungesättigte Fettsäuren und andere.
e) Höhere Fettalkohole, vorzugsweise solche mit wenigstens 12 Kohlenstoffatomen, beispielsweise Laurylalkohol, Cetylalkohol, Stearylalkohol, Oleylalkohol, Behenylalkohol, Cholesterol und/oder 2-Hexadecanaol und andere.
f) Ester, vorzugsweise solche wie Cetyloctanoat, Lauryllactat, Myristyllactat, Cetyllactate, Isopropylmyristat, Myristylmyristat, lsopropylpalmitat, Isopropyladipat, Butylstearat, Decyl oleat, Cholesterol isostearat, Glycerol monostearat, Glyceroldistearat, Glyceroltristearat, Alkyllactat, Alkylcitrat und/oder Alkyltartrat und andere.
g) Lipide wie beispielsweise Cholesterol, Ceramide und/oder Saccharoseester und andere.
h) Vitamine wie beispielsweise die Vitamine A und E, Vitaminalkylester, einschließlich Vitamin C Alkylester und andere.
i) Sonnenschutzmittel
j) Phospholipide
k) Derivate von alpha-Hydroxysäuren
l) Germizide für den kosmetischen Gebrauch, sowohl synthetische wie beipielsweise Salicylsäure und/oder andere als auch natürliche wie beispielsweise Neemöl und/oder andere.
m) Silikone
n) Mischungen jeglicher vorgenannter Komponenten.

Ein weiteres Anwendungsgebiet, insbesondere für erfindungsgemäße Mittel mit kationischen Emulgatoren, ist die Beduftung von, vorteilhafterweise wässrigen, Textilbehandlungsmitteln, vorzugsweise Textilnachbehandlungsmitteln, z.B. von Wäscheweichspülmitteln. Aber auch die Parfümierung von Textilbehandlungsflotten selbst kann mit Hilfe der erfindungsgemäßen Parfumölkonzentrate erfolgen. Die emulgierten Parfumöle können auch in flüssige Waschmittel einemulgiert werden. Schließlich kann man die Parfumölkonzentrate für alle Aufgaben der Beduftung technischer und kosmetischer Produkte verwenden, insbesondere dort, wo man keine alkoholischen Zubereitungen einsetzen kann.

Möglich ist daher ein Erzeugnis zur Textilbehandlung, enthaltend ein erfindungsgemäßes Mittel und einen für die Textilbehandlung geeigneten Stoff.

In einer bevorzugten Ausführungsform enthält ein solches Erzeugnis
a) 0,01 bis 50 Gew.-% eines erfindungsgemäßen Mittels, sowie
b) 0,1 bis 50 Gew.-% eines zur Textilbehandlung geeigneten Stoffes, vorzugsweise Waschtensids, vorteilhafterweise ausgewählt aus der Gruppe nichtionisches, anionisches, kationisches, zwitterionisches und/oder ampholytisches Waschtensid, und
c) gegebenenfalls und vorzugsweise bis zu 50 Gew.-% eines oder mehrerer Waschadditive, vorteilhafterweise ausgewählt aus Buildern, Enzymen, Aufhellern, Schmutzabweisemitteln, Schaumreguliermitteln, antistatischen Mitteln und/oder Dispergiermitteln.
wobei die Gew.-% Angabe jeweils auf das gesamte Erzeugnis bezogen ist.

Die erfindungsgemäßen Mittel lassen sich auch problemlos versprühen. Vorteilhafterweise lassen sie sich auch auf feste, beispielsweise pulverförmige oder granulare, Waschmittel aufsprühen. Besonders vorteilhaft ist dabei, daß die Tröpfchengröße des aufgesprühten Parfumöls um ca. eine Zehnerpotenz kleiner ist als die der originären Parfumöle. So werden die Mittel besonders gut von dem festen Waschmittel aufgenommen.

### Beispiele

### Herstellung von emulgiertem Parfumöl

### Beispiel 1:

### Herstellung von 100 g Emulsion mit einem Gehalt an Parfumöl von 64,4 Gew.-%.

0,35 g Hydroxyethylcellulose (Natrosol®: Bezugsquelle: Hercules Aqualon) wurden in 35 g Wasser bei 25°C unter Rühren im Becherglas 1 gelöst. Im Becherglas 2 erfolgte die Zugabe von 0,25 g Eumulgin® B3 (Cetylstearylalkohol+30-EO; Bezugsquelle: Cognis Deutschland GmbH) zu 64,4 g kommerziell erhältlichem Parfumöl unter Rühren und Aufheizen auf etwa 45°C, bis eine weitestgehend klare Lösung erhalten wurde. Das Gemisch im Becherglas 2 ließ man auf 30°C abkühlen und gab dann innerhalb von 20 Sekunden unter Einsatz eines Laborhomogenisierstabs vom Typ Ultraturrax (Janke und Kunkel) diese Lösung in die wässrige Phase (Becherglas 1). Anschließend erfolgte die Homogenisierung des gesamten Gemisches bei maximaler Drehzahl für 30 s (Die mittlere Tröpfchengröße der Emulsion d₅₀ beträgt ca 2 Mikrometer).

Die beiden Komponenten Wasser und Parfumöl machen in diesem Beispiel 99,4 Gew.-% des Parfumölkonzentrats aus.

### Beispiel 2:

### Herstellung von 100 g Emulsion mit einem Gehalt an Parfumöl von 50 Gew.-%.

Im Becherglas 1 wurden 0,2 g Xanthan in 49,65 g Wasser gelöst, während im Becherglas 2 die Lösung von 0,15 g Eumulgin® B 3 (Bezugsquelle: Cognis Deutschland GmbH) in 50 g Parfumöl bei 45 °C erfolgte. Nach Abkühlen auf 30 °C wurde der Inhalt des Becherglases 2 in das Becherglas 1 eingerührt. Anschließend ist die Homogenisierung mit dem Ultraturrax bei maximaler Drehzahl für 45 s durchgeführt worden.

### Beispiel 3:

### Herstellung von 100 g Emulsion mit einem Gehalt an Parfümöl von 50 %

Im Becherglas 1 wurden 0,2 g Xanthan in 49,65 g Wasser, im Becherglas 2 0,15 g Eumulgin® C4 (Kokosfettsäuremonoethanolamid+4-EO; Bezugsquelle Cognis Deutschland GmbH) in 50 g Parfümöl bei 45 °C gelöst. Nach Abkühlen auf 35 °C wurde der Inhalt des Becherglases 2 in das Becherglas 1 eingerührt und anschließend mittels Ultraschall emulgiert (Ultraschallstab der Fa. Bandelin, Typ Sonopuls HD 2200 mit SH 225 G). Die Emulsion wies nach Messung mittels Laserbeugung (Fa. Malvern) eine mittlere Tröpfchengröße d₅₀ von 600 nm auf.

Die beiden Komponenten Wasser und Parfumöl machen in den Beispielen 2 und 3 99,65 Gew.-% des Parfumölkonzentrats aus.

### Beispiel 4:

### Herstellung von 1000 g Nanoemulsion mit einem Gehalt an Parfumöl von 50 Gew.-%.

In einem großen Becherglas 1 wurden 463 g Wasser vorgelegt und darin 1 g Xanthan aufgelöst. Im Becherglas 2 erfolgte die Aufheizung von 500 g Parfümöl auf 45 °C. Darin sind 20 g Dehydol® LS 2 (C₁₂-C₁₄-Fettalkohol + 2 EO, Fa. Cognis) und 16 g Eumulgin® B3 suspendiert und gelöst worden. Der Inhalt des Bechers 2 wurde in das Wasser im Becherglas 1 eingerüht und anschließend mit dem Ultraturrax homogenisiert. Diese Lösung diente nach dem Abkühlen auf 30 °C als Ausgangsmaterial für den Hochdruckhomogenisator (Technikumsmaschine der Fa. Nivo/Soavi), der bei einem Druck von 700 bar bei max. 45 °C (über Kühlung geregelt) dreimal durchlaufen wurde. Die Nanoemulsion entsteht bereits im 1. Durchlauf, der 2. und 3. Durchlauf wurde zur Minimierung des Tröpfchenanteils über 200 nm genutzt. Die Emulsion wies nach Messung mittels Laserbeugung (Fa. Malvern) eine mittlere Tröpfchengröße d₅₀ von 110 nm auf. Die kleinsten Tröpfchen lagen bei 50 nm, die größten bei 200 nm. Die Stabilität der Emulsion wurde nach verschiedenen Verfahren überprüft. Nach einem Lagertest bei konstant 50°C über einer Dauer von 7 Tagen war die Mikroemulsion unverändert stabil. Nach einem dreiminütigen Zentrifugentest bei 25°C und einer Zentrifugalbeschleunigung von 36 000 g war die Mikroemulsion unverändert stabil.

Die beiden Komponenten Wasser und Parfumöl machen in diesem Beispiel 96,3 Gew.-% des Parfumölkonzentrats aus.

## Patentansprüche

1. Duftstoff- bzw. Parfumölkonzentrate in Form wässriger Emulsionen, enthaltend wenigstens 30 Gew.-% an Parfumöl(en), **dadurch gekennzeichnet, daß** der Gehalt der beiden Komponenten Wasser und Parfumöl(e) einen Wert von zusammen 96 Gew.-% übersteigt, bezogen auf das gesamte Konzentrat.

2. Mittel nach Anspruch 1, **dadurch gekennzeichnet, daß** der Gehalt der beiden Komponenten Wasser und Parfumöl(e) einen Wert von zusammen 97 Gew.-%, vorzugsweise von 98 Gew.-%, vorteilhafterweise von 99 Gew.-%, insbesondere von 99,5 Gew.-% übersteigt, bezogen auf das gesamte Konzentrat.

3. Mittel nach einem der Ansprüche 1 oder 2, **dadurch gekennzeichnet, daß** die mittlere Tröpfchengröße der Emulsion d₅₀ im wesentlichen größer als 0,1 µm ist und eine Obergrenze von 5 µm im wesentlichen nicht überschreitet.

4. Mittel nach einem der Ansprüche 1 oder 2, **dadurch gekennzeichnet, daß** die die mittlere Tröpfchengröße der Emulsion d₅₀ nicht größer als 400 nm ist.

5. Mittel nach einem der Ansprüche 1 bis 4, **dadurch gekennzeichnet daß** es mindestens 40 Gew.-%, vorzugsweise mindestens 50 Gew.-%, vorteilhafterweise mindestens 53 Gew.-%, insbesondere mindestens 55 Gew.-%, in besonders vorteilhafter Weise mindestens 60 Gew.-% an Parfumöl(en) enthält.

6. Mittel nach einem der Ansprüche 1 bis 5, **dadurch gekennzeichnet, daß** es nicht mehr als 90 Gew.-% an Parfumöl(en) enthält.

7. Mittel nach einem der Ansprüche 1 bis 6, **dadurch gekennzeichnet, daß** es weniger als 60 Gew.-%, vorteilhafterweise weniger als 50 Gew.-%, insbesondere weniger als 40 Gew.-% an Wasser enthält.

8. Mittel nach einem der Ansprüche 1 bis 7, **dadurch gekennzeichnet, daß** es mindestens 0,1 Gew.-%, aber weniger als 4 Gew.-%, vorzugsweise weniger als 2,5 Gew.-%, vorteilhafterweise weniger als 2,0 Gew.-%, in sehr vorteilhafter Weise weniger als 1,5 Gew.-%, in überaus vorteilhafter Weise weniger als 1,0 Gew.-%, insbesondere aber weniger als 0,7 Gew.-% an Emulgator enthält, bezogen auf das gesamte Mittel.

9. Mittel nach Anspruch 8, **dadurch gekennzeichnet, daß** der Emulgator ausgewählt ist aus der Gruppe der nichtionischen, zwitterionischen, ampholytischen, kationischen und/oder anionischen Emulgatoren.

10. Mittel nach einem der Ansprüche 1 bis 9, **dadurch gekennzeichnet, daß** es mindestens 0,1 Gew.-%, aber weniger als 4 Gew.-%, vorzugsweise weniger als 2,5 Gew.-%, vorteilhafterweise weniger als 2,0 Gew.-%, in sehr vorteilhafter Weise weniger als 1,5 Gew.-%, in überaus vorteilhafter Weise weniger als 1,0 Gew.-%, insbesondere aber weniger als 0,7 Gew.-% an Verdickungsmittel enthält, bezogen auf das gesamte Mittel.

11. Mittel nach Anspruch 10, **dadurch gekennzeichnet, daß** das Verdickungsmittel ausgewählt ist aus der Gruppe der
a) Polysaccharide, insbesondere Xanthan-Gum, Guar-Derivate, Gummi ara-bicum, Karaya-Gummi, Traganth, Taragummi, Gellan, Carrageen, Johannisbrotkernmehl, Agar-Agar, Alginate, Pektine und/oder Dextrane,
b) organische vollsynthetische Verdickungsmittel, insbesondere Polyacrylate, Polyacrylamide, Polyvinylpyrrolidon, Polyvinylalkohol,Polyethylenglykole, hydrophob modifizierte Polyether, Polyurethane, Styrol-Maleinsäureanhydrid-Copolymerisate, deren Salze und/oder Derivate,
c) Cellulose-Derivate, insbesondere Hydroxyethylcellulose, Carboxymethylcellulose, Hydroxypropyl-methylcellulose, Hydroxypropylcellulose, Ethylhydroxyethyl-cellulose, Methylcellulose,
d) Stärke-Fraktionen und Derivate, insbesondere Amylose, Amylopektin und Dextrine,
e) Tone, insbesondere Bentonit.

12. Mittel nach einem der Ansprüche 1 bis 11, **dadurch gekennzeichnet, daß** eine Mindestmenge an Emulgator von 0,12 Gew.-%, vorteilhafterweise von 0,2 Gew.-%, jeweils bezogen auf das gesamte Mittel, nicht unterschritten wird.

13. Mittel nach einem der Ansprüche 1 bis 12, **dadurch gekennzeichnet, daß** eine Mindestmenge an Verdickungsmittel von 0,12 Gew.-%, vorteilhafterweise von 0,2 Gew.-%, jeweils bezogen auf das gesamte Mittel, nicht unterschritten wird.

14. Verfahren zur Herstellung eines Mittels aufweisend die Merkmale eines der Ansprüche 1 bis 13, umfassend die Schritte:
a) Zugabe von Verdickungsmittel zu Wasser bei Rührung des Gemisches in einem ersten Gefäß,
b) Zugabe von Emulgator zum Parfumöl unter Rührung in einem zweiten Gefäß,
c) Zugabe des Inhalts des zweiten Gefäßes in das Gemisch des ersten Gefäßes unter Einsatz einer Homogenisiermaschine.

15. Verfahren nach Anspruch 14, **dadurch gekennzeichnet daß** die Zugabe des Emulgators zum Parfumöl bei Temperaturen unterhalb 60°C, vorteilhafterweise im Temperaturbereich von 25 bis 55°C erfolgt.

16. Verfahren nach einem der Ansprüche 14 oder 15 , **dadurch gekennzeichnet, daß** das Gemisch des ersten Gefäßes vor der Parfumöl/Emulgator-Zugabe auf eine Temperatur unterhalb 50°C, vorzugsweise unterhalb 40°C, vorteilhafterweise auf eine Temperatur im Bereich von 20-35°C temperiert wird.

17. Verfahren zur Herstellung eines Mittels aufweisend die Merkmale eines der Ansprüche 1 bis 13, **dadurch gekennzeichnet, daß** es in einem einzigen Gefäß durchgeführt wird, wobei zuerst das Wasser vorgelegt wird, danach das Verdickungsmittel und der Emulgator, vorteilhafterweise gleichzeitig, unter Rührung zugegeben werden, und anschließend das Parfumöl unter Einsatz einer Homogenisiermaschine zugegeben wird.

18. Verfahren nach Anspruch 17, **dadurch gekennzeichnet, daß** die Zugabe des Verdickungsmittels bei Temperaturen unterhalb 60°C, vorzugsweise unterhalb 50°C, vorteilhafterweise bei Temperaturen im Bereich von 15-30°C erfolgt und daß die Zugabe des Emulgators während oder nach der Temperierung des Gemisches auf eine Temperatur unterhalb 70°C, vorzugsweise unterhalb 60°C, vorteilhafterweise bei Temperaturen im Bereich 35-55°C erfolgt und daß das Gemisch vor der Parfumölzugabe auf eine Temperatur unterhalb 50°C, vorzugsweise unterhalb 40°C, vorteilhafterweise auf eine Temperatur im Bereich von 20-35°C abgekühlt wird.

19. Verfahren nach einem der Ansprüche 14 bis 18, **dadurch gekennzeichnet, daß** es unter Gaszugabe erfolgt, wobei das Gas aus der Gruppe der Edelgase, Stickstoff und/oder Kohlendioxid ausgewählt ist.

## Claims

1. Odoriferous substance or perfume oil concentrates in the form of aqueous emulsions, containing at least 30% by weight of perfume oil(s), **characterized in that** the content of both components, water and perfume oil(s), exceed a value of together 96% by weight, based on the whole concentrate.

2. The agent according to claim 1, **characterized in that** the content of both components, water and perfume oil(s), exceed a value of together 97% by weight, preferably 98% by weight, advantageously 99% by weight, in particular 99.5% by weight, based on the whole concentrate.

3. The agent according to any of claims 1 or 2, **characterized in that** the average droplet size of the emulsion d₅₀ is essentially larger than 0.1 µm and does not substantially exceed an upper limit of 5 µm.

4. The agent according to any of claims 1 or 2, **characterized in that** the average droplet size of the emulsion d₅₀ is not larger than 400 nm.

5. The agent according to any of claims 1 to 4, **characterized in that** it contains at least 40% by weight, preferably at least 50% by weight, advantageously at least 53% by weight, in particular at least 55% by weight, most preferably at least 60% by weight of perfume oil(s).

6. The agent according to any of claims 1 to 5, **characterized in that** it does not contain more than 90% by weight of perfume oil(s).

7. The agent according to any of claims 1 to 6, **characterized in that** it contains less than 60% by weight, advantageously less than 50% by weight, in particular less than 40% by weight of water.

8. The agent according to any of claims 1 to 7, **characterized in that** it contains at least 0.1 % by weight, but less than 4% by weight, preferably less than 2.5% by weight, advantageously less than 2.0% by weight, most advantageously less than 1.5% by weight, even more advantageously less than 1.0% by weight, in particular however less than 0.7% by weight of emulsifier, based on the whole agent.

9. The agent according to claim 8, **characterized in that** the emulsifier is selected from the group of non-ionic, zwitterionic, ampholytic, cationic and/or anionic emulsifiers.

10. The agent according to any of claims 1 to 9, **characterized in that** it contains at least 0.1 % by weight, but less than 4% by weight, preferably less than 2.5% by weight, advantageously less than 2.0% by weight, most advantageously less than 1.5% by weight, even more advantageously less than 1.0% by weight, in particular however less than 0.7% by weight, of thickener, based on the whole agent.

11. The agent according to claim 10, **characterized in that** the thickener is selected from the group of
a) polysaccharides, in particular xanthan gum, guar derivatives, gum arabic, karaya gum, tragacanth gum, tara gum, gellan, carragheen, carob seed grain, agar, alginates, pectins and/or dextrans,
b) organic fully synthetic thickeners, in particular polyacrylates, polyacrylamides, polyvinylpyrrolidone, polyvinyl alcohol, polyethylene glycols, hydrophobic modified polyethers, polyurethanes, styrene-maleic acid anhydride copolymers, their salts and/or derivatives,
c) cellulose derivatives, in particular hydroxyethylcellulose, carboxymethylcellulose, hydroxypropylmethylcellulose, hydroxypropylcellulose, ethylhydroxyethylcellulose, methylcellulose,
d) starch fractions and derivatives, in particular amylose, amylopectin, and dextrins,
e) clays, in particular bentonite.

12. The agent according to any of claims 1 to 11, **characterized in that** a minimum amount of emulsifier of 0.12% by weight, advantageously 0.2% by weight, in each case based on the whole agent, is not exceeded.

13. The agent according to any of claims 1 to 12, **characterized in that** a minimum amount of thickener of 0.12% by weight, advantageously 0.2% by weight, in each case based on the whole agent, is not exceeded.

14. A method for making an agent having the characteristics of any of claims 1 to 13, comprising the steps of:
a) adding a thickener to water while stirring the mixture in a first container,
b) adding an emulsifier to the perfume oil with stirring in a second container,
c) adding the contents of the second container into the mixture of the first container with the use of a homogenizer machine.

15. The method according to claim 14, **characterized in that** adding the emulsifier to the perfume oil occurs at temperatures below 60°C, advantageously in the temperature range from 25 to 55°C.

16. The method according to any of claims 14 or 15, **characterized in that** a mixture of the first container is temperature-equalized at a temperature below 50°C, preferably below 40°C, advantageously at a temperature in the 20-35°C range, before adding the perfume oil/emulsifier.

17. A method for preparing an agent having the characteristics of any of claims 1 to 13, **characterized in that** it is performed in a single container, in which water is placed first, and the thickener and the emulsifier are then added, preferably simultaneously with stirring and subsequently the perfume oil is added by using a homogenizer machine.

18. The method according to claim 17, **characterized in that** adding the thickener occurs at temperatures below 60°C, preferably below 50°C, advantageously at temperatures in the 15-30°C range, and **in that** adding the emulsifier occurs during or after temperature equalization of the mixture to a temperature below 70°C, preferably below 60°C, advantageously to temperatures in the 35-55°C range, and that the mixture is cooled to a temperature below 50°C, preferably below 40°C, advantageously to a temperature in the 20-35°C range, before adding the perfume oil.

19. The method according to any of claims 14 to 18, **characterized in that** it occurs with addition of gas, the gas being selected from the group of noble gases, nitrogen and/or carbon dioxide.

## Revendications

1. Concentrats de substance(s) odoriférante(s) ou d'huile(s) parfumée(s) sous forme d'émulsions aqueuses, contenant au moins 30% en poids d'huile(s) parfumée(s), **caractérisés, en ce que** la teneur des deux composants eau et huile(s) parfumée(s) est supérieure à une valeur d'au total 96% en poids, par rapport à la totalité du concentrat.

2. Agent selon la revendication 1, **caractérisé en ce que** la teneur des deux composants eau et huile(s) parfumée(s) est supérieure à une valeur d'au total 97% en poids, de préférence supérieure à une valeur d'au total 98% en poids, avantageusement supérieure à une valeur d'au total 99% en poids, en particulier supérieure à une valeur d'au total 99,5% en poids, par rapport à la totalité du concentrat.

3. Agent selon l'une quelconque des revendications 1 ou 2, **caractérisé en ce que** la grosseur moyenne des gouttes de l'émulsion d₅₀ est essentiellement supérieure à 0,1 µm et ne dépasse essentiellement pas une limite supérieure de 5 µm.

4. Agent selon l'une quelconque des revendications 1 ou 2, **caractérisé en ce que** la grosseur moyenne des gouttes de l'émulsion d₅₀ n'est pas supérieure à 400 nm.

5. Agent selon l'une quelconque des revendications 1 à 4, **caractérisé en ce qu'**il contient au moins 40% en poids, de préférence au moins 50% en poids, avantageusement au moins 53% en poids, en particulier au moins 55% en poids, de manière particulièrement avantageuse au moins 60% en poids d'huile(s) parfumée(s).

6. Agent selon l'une quelconque des revendications 1 à 5, **caractérisé en ce qu'**il ne contient pas plus de 90% en poids d'huile(s) parfumée(s).

7. Agent selon l'une quelconque des revendications 1 à 6, **caractérisé en ce qu'**il contient moins de 60% en poids, avantageusement moins de 50% en poids, en particulier moins de 40% en poids d'eau.

8. Agent selon l'une quelconque des revendications 1 à 7, **caractérisé en ce qu'**il contient au moins 0,1% en poids, mais moins de 4% en poids, de préférence moins de 2,5% en poids, avantageusement moins de 2,0% en poids, de manière très avantageuse moins de 1,5% en poids, de manière tout à fait avantageuse moins de 1,0% en poids, en particulier cependant moins de 0,7% en poids d'émulsifiant, par rapport à la totalité de l'agent.

9. Agent selon la revendication 8, **caractérisé en ce que** l'émulsifiant est choisi dans le groupe des émulsifiants non ioniques, zwittérioniques, ampholytiques, cationiques et/ou anioniques.

10. Agent selon l'une quelconque des revendications 1 à 9, **caractérisé en ce qu'**il contient au moins 0,1% en poids, mais moins de 4% en poids, de préférence moins de 2,5% en poids, avantageusement moins de 2,0% en poids, de manière très avantageuse moins de 1,5% en poids, de manière tout à fait avantageuse moins de 1,0% en poids, en particulier cependant moins de 0,7% en poids d'épaississant, par rapport à la totalité de l'agent.

11. Agent selon la revendication 10, **caractérisé en ce que** l'épaississant est choisi dans le groupe formé par
a) les polysaccharides, en particulier la gomme de xanthane, les dérivés de guar, la gomme arabique, la gomme de karaya, la gomme adragante, la gomme tara, le gellan, le carragheen, la farine de graines de caroube, l'agar-agar, les alginates, les pectines et/ou les dextranes,
b) les épaississants organiques complètement synthétiques, en particulier les polyacrylates, les polyacrylamides, la polyvinylpyrrolidone, le poly(alcool vinylique), les polyéthylèneglycols, les polyéthers modifiés de manière hydrophobe, les polyuréthanes, les copolymères de styrène-anhydride d'acide maléique, leurs sels et/ou leurs dérivés,
c) les dérivés de cellulose, en particulier l'hydroxyéthylcellulose, la carboxyméthylcellulose, l'hydroxypropylméthylcellulose, l'hydroxypropylcellulose, l'éthylhydroxyéthylcellulose, la méthylcellulose,
d) les fractions d'amidon et leurs dérivés, en particulier l'amylose, l'amylopectine et les dextrines,
e) les argiles, en particulier la bentonite.

12. Agent selon l'une quelconque des revendications 1 à 11, **caractérisé en ce que** la quantité minimale d'émulsifiant n'est pas inférieure à 0,12% en poids, avantageusement pas inférieure à 0,2% en poids, à chaque fois par rapport à la totalité de l'agent.

13. Agent selon l'une quelconque des revendications 1 à 12, **caractérisé en ce que** la quantité minimale d'épaississant n'est pas inférieure à 0,12% en poids, avantageusement pas inférieure à 0,2% en poids, à chaque fois par rapport à la totalité de l'agent.

14. Procédé pour la préparation d'un agent présentant les caractéristiques selon l'une quelconque des revendications 1 à 13, comprenant les étapes :
a) addition d'épaississant à l'eau sous agitation du mélange dans un premier récipient,
b) addition de l'émulsifiant à l'huile parfumée sous agitation dans un deuxième récipient,
c) addition du contenu du deuxième récipient dans le mélange du premier récipient avec utilisation d'une machine d'homogénéisation.

15. Procédé selon la revendication 14, **caractérisé en ce que** l'addition de l'émulsifiant à l'huile parfumée est réalisée à des températures inférieures à 60°C, avantageusement dans la plage de température de 25 à 55°C.

16. Procédé selon l'une quelconque des revendications 14 ou 15, **caractérisé en ce que** le mélange du premier récipient, avant l'addition de l'huile parfumée/émulsifiant, est équilibré thermiquement à une température inférieure à 50°C, de préférence inférieure à 40°C, avantageusement à une température dans la plage de 20-35°C.

17. Procédé pour la préparation d'un agent présentant les caractéristiques selon l'une quelconque des revendications 1 à 13, **caractérisé en ce qu'**il est réalisé dans un seul récipient, en disposant au préalable d'abord l'eau, puis en y ajoutant l'épaississant et l'émulsifiant, avantageusement en même temps, sous agitation, puis en ajoutant l'huile parfumée, avec utilisation d'une machine d'homogénéisation.

18. Procédé selon la revendication 17, **caractérisé en ce que** l'addition de l'épaississant est réalisée à des températures inférieures à 60°C, de préférence inférieures à 50°C, avantageusement à des températures dans la plage de 15-30°C et **en ce que** l'addition de l'émulsifiant est réalisée pendant ou après l'équilibrage thermique du mélange à une température inférieure à 70°C, de préférence inférieure à 60°C, avantageusement à des températures dans la plage de 35-55°C et **en ce que** le mélange est refroidi, avant l'addition d'huile parfumée, à une température inférieure à 50°C, de préférence inférieure à 40°C, avantageusement à une température dans la plage de 20-35°C.

19. Procédé selon l'une quelconque des revendications 14 à 18, **caractérisé en ce qu'**il est réalisé sous addition de gaz, le gaz étant choisi dans le groupe des gaz nobles, de l'azote et/ou du dioxyde de carbone.
